# EUROPEAN PATENT APPLICATION

(11) **EP 3 605 457 A1**
(43) Date of publication of application: **05.02.2020**
(21) Application number: 18775568.1
(22) Date of filing: 16.02.2018
(51) Int. Cl.: G06T 7/00, C12M 1/34, G06T 7/62

(54) **INFORMATION PROCESSING DEVICE, INFORMATION PROCESSING METHOD, PROGRAM, AND OBSERVATION SYSTEM**

(30) Priority: 31.03.2017 JP 2017072857
(71) Applicant: Sony Corporation, Tokyo 108-0075 (JP)
(72) Inventor: SHINODA, Masataka, Tokyo 108-0075 (JP); OHASHI, Takeshi, Tokyo 108-0075 (JP)
(74) Representative: D Young & Co LLP
(86) International application number: PCT/JP2018/005462
(87) International publication number: WO 2018/179971

(57) **Abstract**

An information processing device according to an embodiment of the present technology includes an image acquisition unit, a feature amount calculation unit, and a determination unit. The image acquisition unit acquires a time lapse image of a fertilized egg imaged on a time-series basis. The feature amount calculation unit calculates a time-series state variation of the fertilized egg. The determination unit determines any one or both of an active period and a rest period of the fertilized egg on the basis of the state variation.

## Description

### Technical Field

The present technology relates to an information processing device, an information processing method, a program, and an observation system which are applicable to evaluation of cells.

### Background Art

In an image processing device described in Patent Literature 1, a reference image is selected from an image group obtained by imaging a plurality of fertilized eggs, and contours of the fertilized eggs are detected as a reference contour. A predetermined profile is executed on the basis of the reference contour to determine contours of fertilized eggs in another arbitrary image of the image group. According to this, it is possible to output a fertilized egg image in which positions of the fertilized eggs are accurately combined to all images of an image group, and improvement of analysis accuracy of the fertilized eggs is also realized.

### Citation List

### Patent Literature

Patent Literature 1: JP-A-2011-192109

### Disclosure of Invention

### Technical Problem

As such, there is a demand for a technology of evaluating a fertilized egg and the like as an observation target with high accuracy.

In consideration of such circumstances, an object of the present technology is to provide an information process device, an information processing method, a program, and an observation system which are capable of evaluating a fertilized egg as an observation target with high accuracy.

### Solution to Problem

To achieve the object, according to an aspect of the present technology, there is provided an information processing device including an image acquisition unit, a feature amount calculation unit, and a determination unit.

The image acquisition unit acquires a time lapse image of a fertilized egg imaged on a time-series basis.

The feature amount calculation unit calculates a time-series state variation of the fertilized egg.

The determination unit determines any one or both of an active period and a rest period of the fertilized egg on the basis of the state variation.

According to the technology, a lag-phase (an activity rest period), which becomes an index when selecting a fertilized egg of developmental potency after transplantation is predicted to be high, is automatically determined. According to this, it is possible to evaluate a fertilized egg before transplantation with high accuracy.

The feature amount calculation unit may calculate a variation of a movement amount inside the fertilized egg as the state variation.

According to this, when the variation of the movement amount inside the fertilized egg is visualized with a graph or the like, in a case where an external shape variation of the fertilized egg is small, it is possible to evaluate performance inside the fertilized egg.

The feature amount calculation unit may further calculate a shape variation of the fertilized egg as the state variation.

According to this, when the shape variation is visualized with a graph or the like, it is possible to confirm a time at which the shape of the fertilized egg starts to vary, a growth speed, or the like, and it is possible to quantitively and intuitively understand contraction activity of the fertilized egg.

The feature amount calculation unit may calculate a diameter variation of the fertilized egg as the shape variation.

The determination unit may determine, as the rest period, a state of the fertilized egg in which the variation of the movement amount per unit time and the shape variation per unit time are in predetermined variation amount ranges.

The determination unit may determine, as the rest period, a state of the fertilized egg in which the shape variation per unit time is approximately zero and the variation of the movement amount per unit time is approximately zero.

The information processing device may further include a prediction unit that calculates, on the basis of any one or both of the active period and the rest period, at least one of a hatching rate, an implantation rate, a pregnancy rate, a conception rate, an abortion rate, a birth weight, a birth rate, or a breeding value relating to the fertilized egg.

According to this, efficiency of a process of selecting a fertilized egg of which the developmental potency after transplantation is predicted to be high is significantly improved.

The prediction unit may calculate at least one of the hatching rate, the implantation rate, the pregnancy rate, the conception rate, the abortion rate, the birth weight, the birth rate, or the breeding value by using a learned model obtained by causing a machine learning algorithm to learn learning data.

To achieve the above-described object, according to another aspect of the present technology, there is provided an information processing method, including:
acquiring a time lapse image of a fertilized egg imaged on a time-series basis;
calculating a time-series state variation of the fertilized egg; and
determining any one or both of an active period and a rest period of the fertilized egg on the basis of the state variation.

To achieve the above-described object, according to still another aspect of the present technology, there is provided a program that causes an information processing device to execute:
a step of acquiring a time lapse image of a fertilized egg imaged on a time-series basis;
a step of calculating a time-series state variation of the fertilized egg; and
a step of determining any one or both of an active period and a rest period of the fertilized egg on the basis of the state variation.

To achieve the above-described object, according to still another aspect of the present technology, there is provided an observation system including an imaging unit and an information processing device.

The imaging unit images a fertilized egg on a time-series basis.

The information processing device includes an image acquisition unit that acquires a time lapse image captured by the imaging unit, a feature amount calculation unit that calculates a time-series state variation of the fertilized egg, and a determination unit that determines any one or both of an active period and a rest period of the fertilized egg on the basis of the state variation.

### Advantageous Effects of Invention

As described above, according to the present technology, it is possible to provide an information processing device, an information processing method, a program, and an observation system which are capable of evaluating a fertilized egg as an observation target with high accuracy. Note that, the effect is not limited, and any one effect described in this specification or another effect capable of being understood from this specification may be exhibited in combination with the above-described effect or instead of the above-described effect.

### Brief Description of Drawings

[Fig. 1] Fig. 1 is a schematic view illustrating a configuration example of an observation system according to a first embodiment of the present technology.
[Fig. 2] Fig. 2 is a schematic view when viewing a culture container group provided on an observation stage of the observation system from a light source side.
[Fig. 3] Fig. 3 is a view schematically illustrating a cross-section of a culture container according to the embodiment.
[Fig. 4] Fig. 4 is a schematic view when viewing the culture container from the light source side.
[Fig. 5] Fig. 5 is a schematic view when viewing a photographing area in the culture container from the light source side.
[Fig. 6] Fig. 6 is a block diagram illustrating a configuration example of the observation system.
[Fig. 7] Fig. 7 is a flowchart illustrating a method of evaluating quality of a fertilized egg in an information processing device according to the embodiment.
[Fig. 8] Fig. 8 is a schematic view illustrating a state in which an imaging unit of the observation system images a plurality of the fertilized eggs.
[Fig. 9] Fig. 9 is a conceptual diagram that virtually illustrates a first time lapse image.
[Fig. 10] Fig. 10 is a conceptual diagram that virtually illustrates a second time lapse image.
[Fig. 11] Fig. 11 is a graph illustrating a state variation of the fertilized eggs.
[Fig. 12] Fig. 12 is a graph illustrating a first feature amount of the fertilized eggs.
[Fig. 13] Fig. 13 is a schematic view in which a second feature amount of the fertilized eggs is visualized according to a second embodiment of the present technology.
[Fig. 14] Fig. 14 is a graph illustrating a variation of a movement amount inside the fertilized eggs according to a third embodiment of the present technology.
[Fig. 15] Fig. 15 is a view illustrating a morphological variation in a growth process of the fertilized eggs.
[Fig. 16] Fig. 16 is a view illustrating a graph that illustrates a state variation of the fertilized eggs and a graph that illustrates a variation of a movement amount inside the fertilized eggs in combination according to a fifth embodiment of the present technology.
[Fig. 17] Fig. 17 is a schematic view illustrating a configuration example of an observation system according to a sixth embodiment of the present technology.
[Fig. 18] Fig. 18 is a block diagram illustrating a procedure of a typical specified AI in a simple manner. Mode(s) for Carrying Out the Invention
Hereinafter, embodiments of the present technology will be described with reference to the accompanying drawings. In the drawings, an X-axis, a Y-axis, and a Z-axis which are orthogonal to each other are illustrated. The X-axis, the Y-axis, and the Z-axis are common to all of the drawings.

### <First Embodiment>

### [Configuration of Observation System]

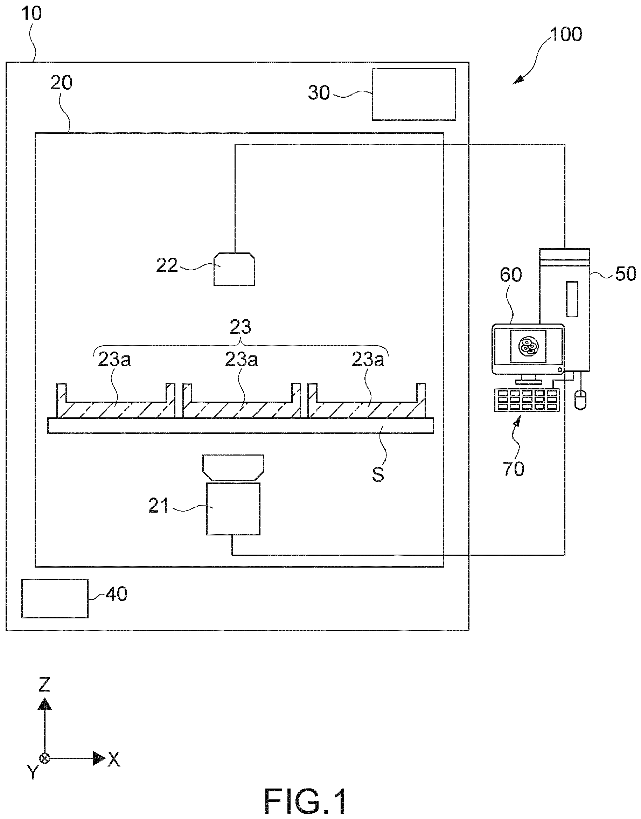

Fig. 1 is a schematic view illustrating a configuration example of an observation system 100 according to a first embodiment of the present technology. As illustrated in Fig. 1, the observation system 100 includes an incubator 10, an observation device 20, a humidity/temperature/gas control unit 30, a detection unit 40, an information processing device 50, a display device 60, and an input unit 70.

The incubator 10 is a culture device that accommodates the observation device 20, the humidity/temperature/gas control unit 30, and the detection unit 40, and has a function of constantly maintaining a temperature, humidity, and the like inside thereof. An arbitrary gas can flow into the incubator 10. A kind of the gas is not particularly limited, and examples thereof include nitrogen, oxygen, carbon dioxide, and the like.

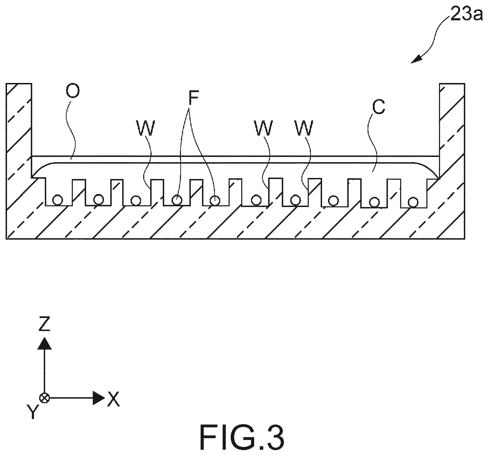

The observation device 20 includes an imaging unit 21, a light source 22, and a culture container group 23. The imaging unit 21 is configured to image fertilized eggs F accommodated in a culture container 23a (dish) (refer to Fig. 3) on a time-series basis and to generate images of the fertilized eggs F.

The imaging unit 21 includes a lens barrel including a lens group capable of moving in an optical axis direction (Z-axis direction), a solid-state imaging element such as a complementary metal oxide semiconductor (CMOS) and a charge coupled device (CCD) which images subject light that passes through the lens barrel, a drive circuit that drives the constituent elements, and the like.

The imaging unit 21 has a configuration capable of moving in the optical axis direction (Z-axis direction) and a horizontal direction (a direction orthogonal to the Z-axis direction), and images the fertilized eggs F accommodated in the culture container 23a while moving in the horizontal direction. In addition, the imaging unit 21 may be configured to photograph not only a still image but also a moving image.

Typically, the imaging unit 21 according to this embodiment is a visible light camera, but may be an infrared (IR) camera, a polarization camera, or the like without limitation to the visible light camera.

When imaging the fertilized eggs F inside the culture container 23a, the light source 22 emits light to the culture container 23a when imaging the fertilized egg F inside the culture container 23a with the imaging unit 21. As the light source 22, for example, a light emitting diode (LED) that emits specific wavelength light, or the like is employed. In a case where the light source 22 is the LED, for example, a red LED that emits light having a wavelength of 640 nm is employed.

The culture container group 23 includes a plurality of the culture containers 23a, and is provided on an observation stage S between the imaging unit 21 and the light source 22. The observation stage S is configured to allow light emitted from the light source 22 to be transmitted therethrough.

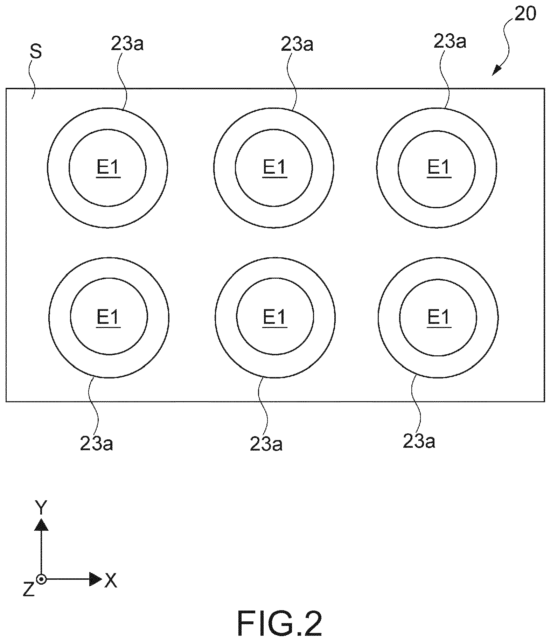

Fig. 2 is a schematic view when viewing the culture container group 23 provided on the observation stage S of the observation device 20 from the light source 22 side. As illustrated in Fig. 2, for example, six pieces of the culture containers 23a are provided on the observation stages S in a matrix shape, and three pieces are provided in the X-axis direction and two pieces are provided in the Y-axis direction.

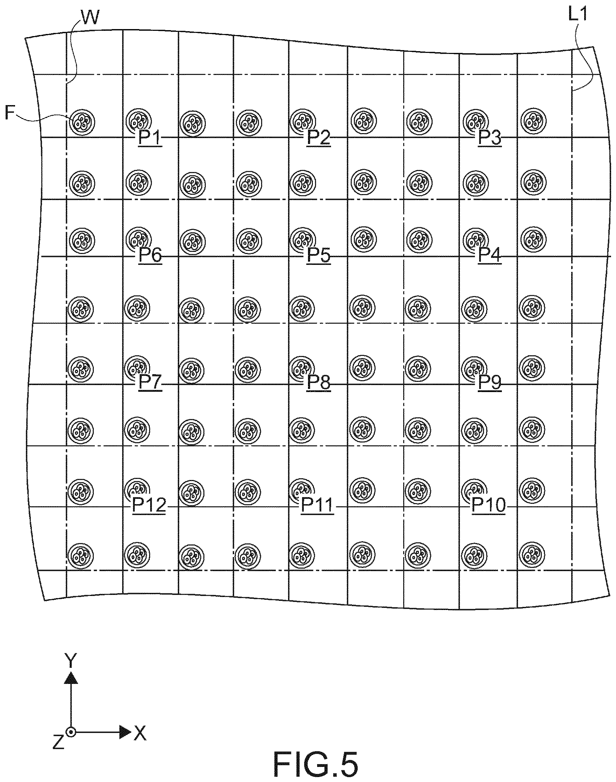

Fig. 3 is a view schematically illustrating a cross-section of each of the culture containers 23a. As illustrated in Fig. 3, a plurality of wells W is provided in the culture container 23a. The wells W are provided in the culture container 23a in a matrix shape (refer to Fig. 5), and each of the wells W is configured to accommodate one piece of the fertilized eggs F.

In addition to the wells W, a culture liquid C and an oil O are injected into the culture container 23a. The culture liquid C is coated with the oil O, and thus the oil O has a function of suppressing evaporation of the culture liquid C.

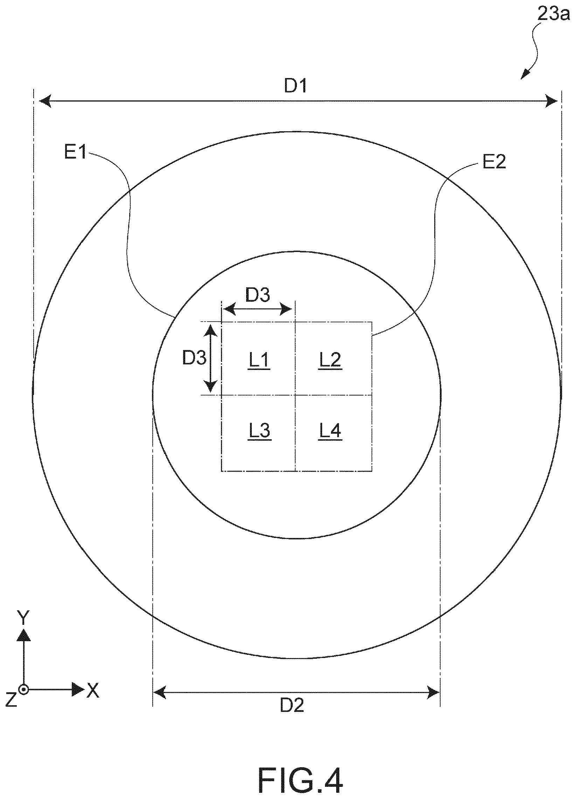

Fig. 4 is a schematic view (plan view) of the culture container 23a that is viewed from the light source 22 side. The culture container 23a includes a well region E1 in which a plurality of wells W is formed. A diameter D1 of the culture container 23a and a diameter D2 of the well region E1 are not particularly limited, and for example, the diameter D1 is approximately 35 mm, and the diameter D2 is approximately 20 mm.

The well region E1 includes a photographing region E2 that becomes a photographing target of the imaging unit 21. As illustrated in Fig. 2, the photographing region E2 is divided into four parts including four photographing areas L1 to L4. For example, a length D3 of one side of each of the photographing areas L1 to L4 is approximately 5 mm.

Fig. 5 is a schematic view illustrating the photographing area L1 viewed from the light source 22 side in an enlarged manner. The photographing area L1 includes seventy two wells W among a plurality of wells W provided in the well region E1, and is divided into twelve parts for every position (POS) region.

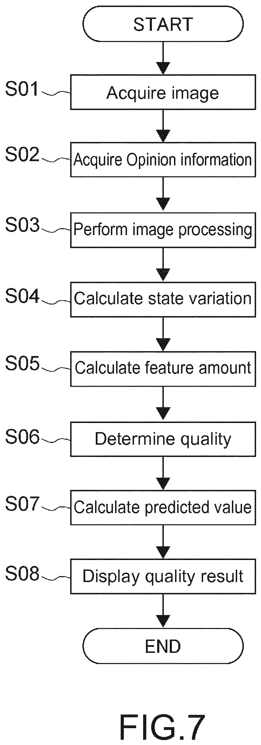

Each of POS regions P1 to P12 includes six wells W which are arranged three (X-axis direction) by two (Y-axis direction). The imaging unit 21 according to this embodiment images the fertilized eggs F which are accommodated in the wells W for every POS region on a time-series basis in an image acquisition process (refer to Fig. 7) to be described later. Note that, Fig. 5 is a schematic view illustrating the photographing area L1 in an enlarged manner, but the photographing areas L2 to L4 have the same configuration as in the photographing area L1.

A material of the culture container 23a is not particularly limited, and examples thereof include inorganic materials such as glass or silicon, organic materials such as a polystyrene resin, a polyethylene resin, a polypropylene resin, an ABS resin, nylon, an acrylic resin, a fluorine resin, a polycarbonate resin, a polyurethane resin, a methyl pentene resin, a phenol resin, a melamine resin, an epoxy resin, and a vinyl chloride resin, and the like, and is a transparent body through which light emitted from the light source 22 is transmitted. Alternatively, in the culture container 23a, a portion other than sites through which the light emitted from the light source 22 is transmitted may be formed from the above-described materials, or a metal material.

The humidity/temperature/gas control unit 30 controls a temperature and humidity inside the incubator 10, and a gas introduced into the incubator 10, and provides an environment suitable for growth of the fertilized eggs F. For example, the humidity/temperature/gas control unit 30 can control the temperature inside the incubator 10 to approximately 38°C.

The detection unit 40 is wirelessly connected to the information processing device 50, and is configured to detect a temperature, humidity, and an atmospheric pressure inside the incubator 10, illuminance of the light source 22, or the like, and to output a detection result to the information processing device 50. Examples of the detection unit 40 include a solar panel type or battery type internet of things (IoT), and the like, and the kind thereof does not matter.

The information processing device 50 include hardware such as a central processing unit (CPU), a read only memory (ROM), a random access memory (RAM), and a hard disk drive (HDD) which are necessary for a computer. When the CPU loads a program stored in the ROM or the HDD according to the present technology in the RAM, and executes the program, an operation of each block of the information processing device 50 to be described later is controlled.

For example, the program is installed in the information processing device 50 through various storage media (internal memories). Alternatively, installation of the program may be executed through the Internet. Typically, the information processing device 50 is a personal computer (PC), but may be another arbitrary computer.

The display device 60 is configured to display an image photographed by the imaging unit 21, and the like. For example, the display device 60 is a display device that uses a liquid crystal, an organic electro-luminescence (EL), or the like.

The input unit 70 is an operation device such as a keyboard and a mouse for inputting a user's operation. The input unit 70 may be a touch panel that is constructed integrally with the display device 60.

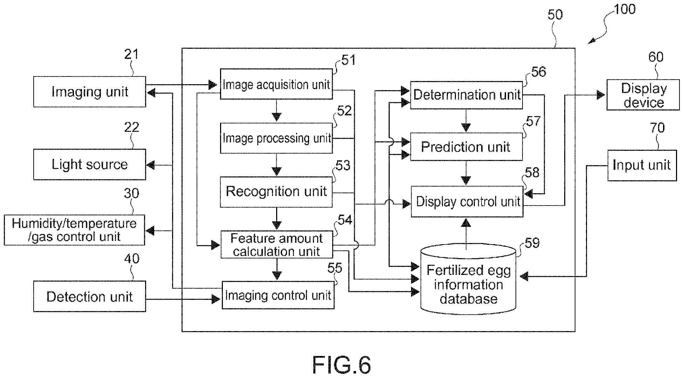

Next, a configuration of the information processing device 50 will be described. Fig. 6 is a block diagram illustrating a configuration example of the observation system 100.

### [Information Processing Device]

As illustrated in Fig. 6, the information processing device 50 includes an image acquisition unit 51, an image processing unit 52, a recognition unit 53, a feature amount calculation unit 54, an imaging control unit 55, a determination unit 56, a prediction unit 57, a display control unit 58, and a fertilized egg information database 59.

The image acquisition unit 51 acquires an image of each of the fertilized eggs F which is captured on a time-series basis by the imaging unit 21. The image processing unit 52 processes (trims) the image acquired from the image acquisition unit 51.

The recognition unit 53 performs predetermined analysis processing with respect to the image acquired from the image acquisition unit 51, and recognizes at least one of a shape of the fertilized egg F or a position of the fertilized egg F in the well W.

The feature amount calculation unit 54 calculates at least one of a time-series state variation of the fertilized egg F or a time-series variation of a relative position of the fertilized egg F with respect to the well W, and calculates at least one of a feature amount (hereinafter, referred to as "first feature amount") based on the state variation, or a feature amount (hereinafter, referred to as "second feature amount") based on the variation of the relative position.

The imaging control unit 55 controls the imaging unit 21 and the light source 22 on the basis of a shape variation (state variation) so that a time at which the fertilized egg F is imaged varies.

For example, the imaging control unit 55 controls the imaging unit 21 and the light source 22 on the basis of numerical value data that is output from the feature amount calculation unit 54 and relates to the shape variation so that a photographing interval when imaging the fertilized egg F is shortened. According to this, it is possible to emit light to the fertilized egg F only when acquiring data having a great influence on quality evaluation of the fertilized egg F. Accordingly, a time for emitting light of the light source 22 to the fertilized egg F as an observation target is shortened, and thus an optical damage (phototoxicity) applied to the fertilized egg F is reduced.

Examples of the optical damage (phototoxicity) include an optical damage or a thermal damage that is applied to a DNA or a chromosome due to light, and the like. The imaging control unit 55 may control the imaging unit 21 and the light source 22 on the basis of not only the shape variation of the fertilized egg F but also an imaging time of the fertilized egg F, a growth stage, or the like.

In addition, the imaging control unit 55 is configured to control the light source 22 and the humidity/temperature/gas control unit 30 on the basis of an output from the detection unit 40. According to this, a temperature and humidity inside the incubator 10, and illuminance of the light source 22 are adjusted.

The determination unit 56 determines quality of the fertilized egg F on the basis of at least one of the first feature amount or the second feature amount.

The prediction unit 57 calculates at least one of a hatching rate, an implantation rate, a pregnancy rate, a conception rate, an abortion rate, a birth weight, a birth rate, or a breeding value relating to the fertilized egg F on the basis of at least one of the state variation, the first feature amount, or the second feature amount which is output from the feature amount calculation unit 54.

The fertilized egg information database 59 stores the image acquired from the image acquisition unit 51, the state variation or the feature amount which is acquired from the feature amount calculation unit 54, input information input from the input unit 70, and the like.

Note that, functions of the image processing unit 52, the recognition unit 53, the feature amount calculation unit 54, the imaging control unit 55, the determination unit 56, the prediction unit 57, the display control unit 58, and the fertilized egg information database 59 are not limited to the above-described functions, and detailed functions thereof will be described in a fertilized egg quality evaluation method to be described later.

### [Fertilized Egg Quality Evaluation Method]

Fig. 7 is a flowchart illustrating a method of evaluating the quality of the fertilized egg F in the information processing device 50. Hereinafter, the method of evaluating the quality of the fertilized egg F will be described appropriately with reference to Fig. 7.

### (Step S01: Image Acquisition)

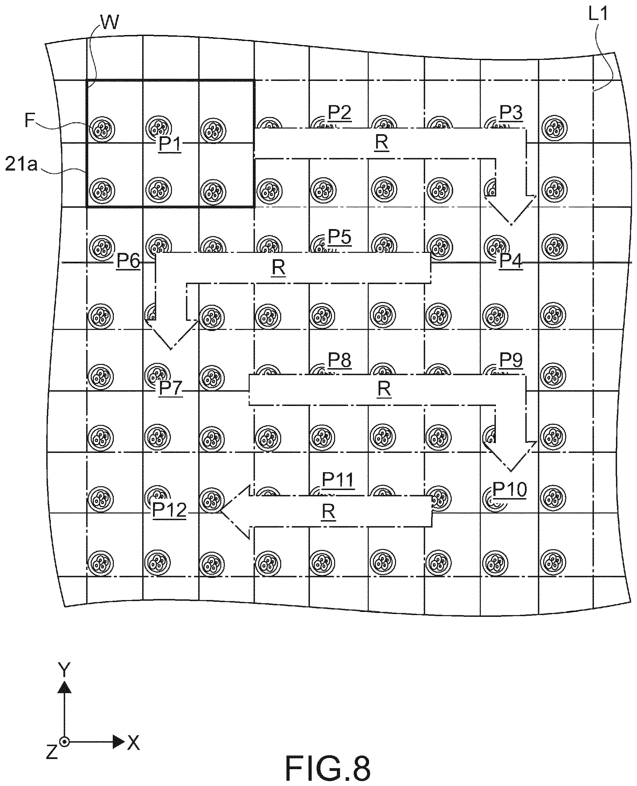

Fig. 8 is a schematic view illustrating a state of imaging a plurality of fertilized eggs F by the imaging unit 21, and is a view illustrating a movement route of the imaging unit 21.

First, the fertilized egg F accommodated in each of a plurality of wells W is imaged by the imaging unit 21 on a time-series basis for every POS (position) region. At this time, as illustrated in Fig. 8, a visual field range 21a of the imaging unit 21 moves along a movement route R sequentially from a POS region P1 to a POS region P12 with an interval of approximately three seconds.

In addition, this process is performed with respect to all of the culture containers 23a provided on the observation stage S, and is repeated specified number of times. According to this, a plurality of images each including six fertilized eggs F (hereinafter, referred to as "first time lapse G1") is generated, and each of the first time lapse images G1 is output to the image acquisition unit 51 (the information processing device 50).

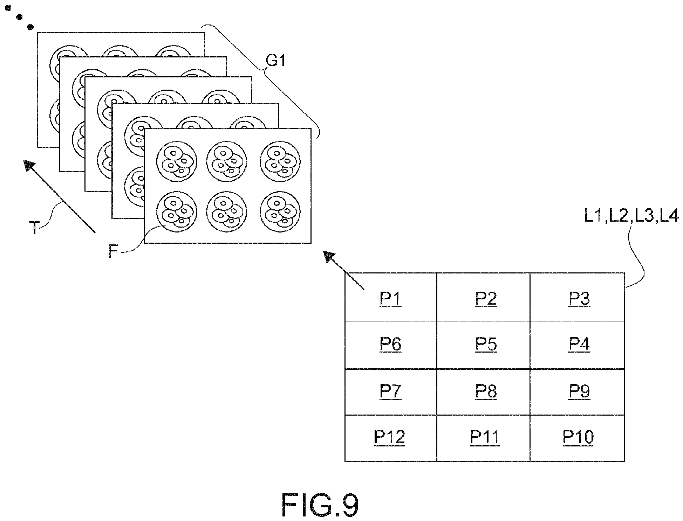

Fig. 9 is a conceptual diagram that virtually illustrates the first time lapse image G1. The first time lapse G1 is generated with respect to the POS regions P1 to P12 on a time-series basis along a time axis T as illustrated in Fig. 9. In this specification, a plurality of pieces of time-series observation image data as illustrated in Fig. 9 is referred to as "first time lapse image G1".

An imaging interval or the number of imaging sheets of the imaging unit 21 in the observation system 100 can be arbitrarily set. For example, in a case where an imaging period is set to one week, the imaging interval is set to fifteen minutes, and nine-stack imaging is performed while changing a focal length in a depth direction (Z-axis direction), approximately 6000 sheets of stacked images each including six fertilized eggs F are obtained with respect to one POS region. According to this, a three-dimensional image of the fertilized eggs F can be acquired.

The image acquisition unit 51 outputs the first time lapse image G1 output from the imaging unit 21 to the image processing unit 52 and the fertilized egg information database 59, and the first time lapse image G1 is stored in the fertilized egg information database 59.

### (Step S02: Opinion Information Acquisition)

The display control unit 58 reads out the first time lapse image G1 stored in the fertilized egg information database 59, and outputs the first time lapse image G1 to the display device 60. According to this, the display device 60 displays the first time lapse image G1.

Next, the first time lapse image G1 displayed on the display device 60 is evaluated by an expert such as an embryologist in accordance with a morphological opinion. According to this, the quality (a growth state, the number of cells, cell symmetry, fragment, and the like) of the fertilized eggs F is evaluated. An evaluation result of the fertilized eggs F evaluated by the embryologist is output to the fertilized egg information database 59 through the input unit 70, and is stored in the fertilized egg information database 59 as first quality data relating to the fertilized eggs F.

Note that, a method of evaluating the quality of the fertilized eggs F by the embryologist is not particularly limited. For example, in step S02, typically, the embryologist evaluates the quality of all of the six fertilized eggs F included in the first time lapse image with respect to the POS regions P1 to P12. However, there is no limitation thereto, and the quality of only a part of the fertilized eggs F may be evaluated. In addition, in the evaluation, all of stacked images per nine stacks with respect to each of the fertilized eggs F may be referred to, or a partial image per nine stacks may be referred to.

### (Step S03: Image Processing)

The image processing unit 52 processes (trims) the first time lapse image G1 acquired from the image acquisition unit 51 in a unit of the fertilized egg F. According to this, an image including one fertilized egg F (hereinafter, referred to as "second time lapse image G2") is generated. Next, the image processing unit 52 outputs the second time lapse image G2 to the recognition unit 53 and the fertilized egg information database 59, and the second time lapse image G2 is stored in the fertilized egg information database 59.

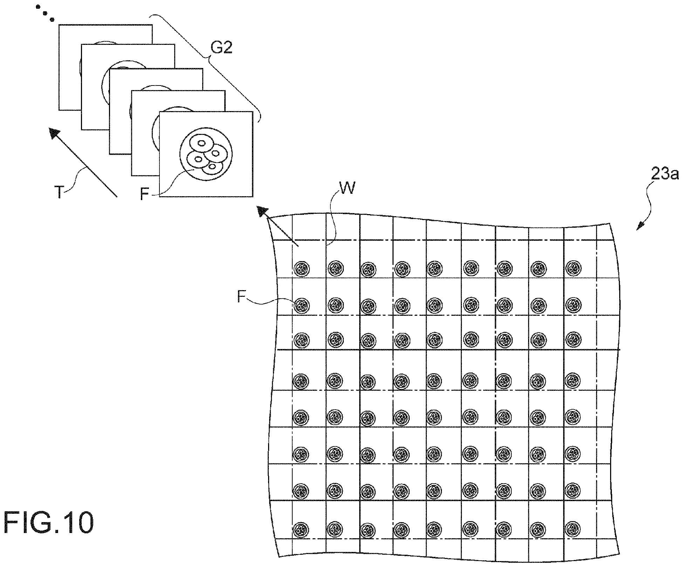

Fig. 10 is a conceptual diagram that virtually illustrates the second time lapse image G2. The second time lapse image G2 is generated with respect to the plurality of wells W on a time-series basis along a time axis T as illustrated in Fig. 10. In this specification, a plurality of pieces of time-series observation image data as illustrated in Fig. 10 is referred to as "second time lapse image G2".

The recognition unit 53 performs predetermined image processing with respect to the second time lapse image G2 acquired from the image processing unit 52. The second time lapse image G2 subjected to the image processing by the recognition unit 53 is output to the feature amount calculation unit 54 and the fertilized egg information database 59, and the second time lapse image G2 is stored in the fertilized egg information database 59.

For example, the recognition unit 53 performs probability processing, binarization processing, overlay processing, and the like by deep learning analysis with respect to the second time lapse image G2 acquired from the image processing unit 52. According to this, for example, a contour line of the fertilized egg F in the second lapse image G2 is extracted.

Alternatively, the recognition unit 53 forms a mask region conforming to a shape of the fertilized egg F with respect to each image that constitutes the second time lapse image G2. According to this, an analysis region (recognition region) of the fertilized egg F in the second time lapse image G2 becomes clear, and thus it is possible to accurately recognize the shape of the fertilized egg F. According to this technology, for example, the recognition unit 53 can accurately recognize a transparent zone that forms an external shape of the fertilized egg F, or a shape of a blastocyst, a cell blastomere, or the like inside the fertilized egg F.

### (Step S04: State Variation Calculation)

The feature amount calculation unit 54 performs predetermined analysis processing with respect to the second time lapse image G2 output from the recognition unit 53 to calculate a time-series shape variation (state variation) of the fertilized egg F (along a time axis T), and outputs numerical value data relating to the shape variation to the imaging control unit 55, the determination unit 56, the prediction unit 57, the display control unit 58, and the fertilized egg information database 59. The numerical value data that relates to the shape variation and is output to the fertilized egg information database 59 is stored in the fertilized egg information database 59.

For example, the feature amount calculation unit 54 calculates a difference value between frames with respect to the second time lapse image G2 output from the recognition unit 53, and calculates the shape variation on the basis of the difference value.

Alternatively, in previous step S03, the feature amount calculation unit 54 may calculate a difference value between a mask region of one image and a mask region of another image with respect to mask regions formed in respective images which constitute the second time lapse G2. That is, the feature amount calculation unit 54 may calculate a difference value between frames of only a mask region conforming to a shape of the fertilized egg F, and may calculate a shape variation of the fertilized egg F on the basis of the difference value.

According to this, occurrence of noise or erroneous detection due to calculation of the difference value between frames in the entirety of the images which constitute the second time lapse image G2 is suppressed, and thus it is possible to accurately calculate the shape variation of the fertilized egg F or a first feature amount to be described later.

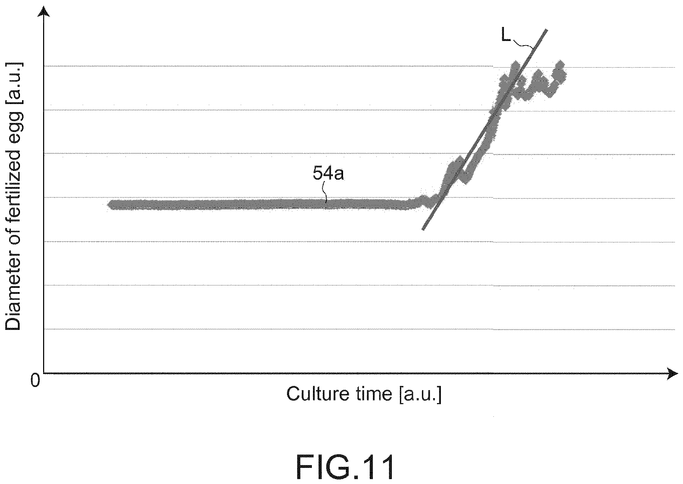

Fig. 11 is a graph illustrating a time-series shape variation (diameter variation) of the fertilized egg F with respect to a culture time. The feature amount calculation unit 54 calculates at least one of time-series variations of a diameter, an area, a volume, or a roundness of the fertilized egg F as the shape variation.

According to this, when illustrating the time-series variations with a graph or the like as illustrated in Fig. 11, it is possible to confirm a time at which the shape of the fertilized egg F starts to vary, a growth speed, or the like, and it is possible to quantitively and intuitively understand contraction activity of the fertilized egg F (for example, a time-series radius variation of the fertilized egg F). Note that, in the example illustrated in Fig. 11, an inclination of a straight line L corresponds to the growth speed of the fertilized egg F.

### (Step S05: Feature Amount Calculation)

Next, the feature amount calculation unit 54 performs predetermined analysis processing such as differential operation with respect to a shape variation that is calculated to calculate a first feature amount of the fertilized egg F, and outputs numerical value data relating to the first feature amount to the imaging control unit 55, the determination unit 56, the prediction unit 57, the display control unit 58, and the fertilized egg information database 59. The numeral value data that is output to the fertilized egg information database 59 and relates to the first feature amount is stored in the fertilized egg information database 59.

In addition, the numeral value data that is output to the fertilized egg information database 59 and relates to the first feature amount is correlated with the first quality data (a growth state, the number of cells, cell symmetry, fragment, and the like) relating to the fertilized egg F having the first feature amount evaluated in previous step S02, and is stored in the fertilized egg information database 59 as second quality data.

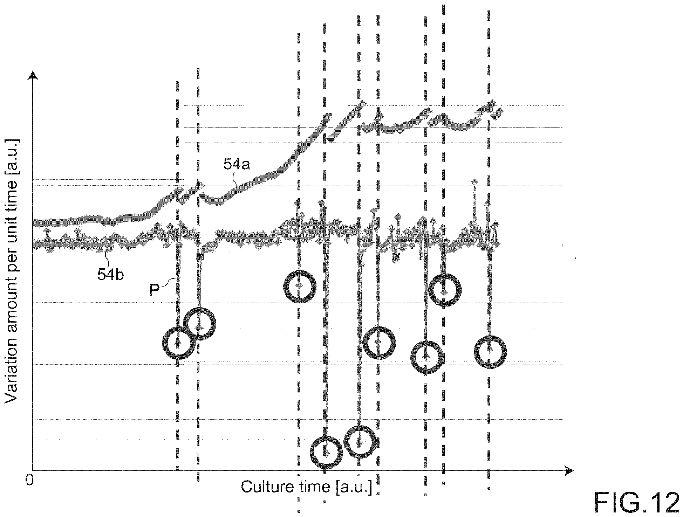

Fig. 12 is a graph illustrating the first feature amount calculated by analyzing a shape variation (diameter variation) of the fertilized egg F. Note that, in Fig. 12, a graph 54a illustrating the shape variation of the fertilized egg F and a graph 54b illustrating the first feature amount are shown in combination.

The feature amount calculation unit 54 calculates at least one of the number of times of contraction, a contraction diameter, a contraction speed, a contraction time, a contraction interval, contraction strength, or a contraction frequency of the fertilized egg F as the first feature amount. According to this, when illustrating these values with a graph or the like as illustrated in Fig. 12, it is possible to quantitively and intuitively understand a minute contraction phenomenon of the fertilized egg F. In the example illustrated in Fig. 12, the number of peaks P detected by analyzing the shape variation of the fertilized egg F corresponds to the number of times of contraction of the fertilized egg F.

In addition, in the present technology, in a case where the feature amount calculation unit 54 calculates a time-series area variation of the fertilized egg F as the shape variation, the first feature amount can also be calculated on the basis of an area difference between the transparent zone of the fertilized egg F which is recognized in previous step S03, and the blastocyst inside the fertilized egg F. For example, the number of times of contraction of each of the transparent zone and the blastocyst is obtained by counting the number of times at which the area difference between the transparent zone and the blastocyst in a culture time of the fertilized egg F becomes zero, and the number of times at which the area difference does not become zero.

### (Step S06: Quality Determination)

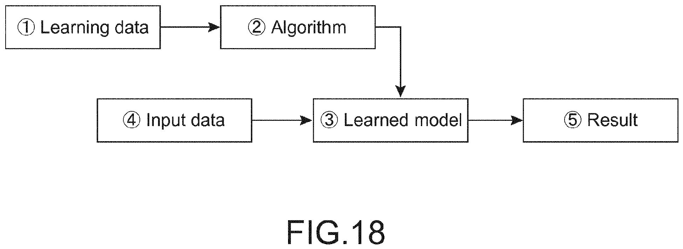

The determination unit 56 determines quality (a growth situation, a quality rank, or the like) of the fertilized egg F on the basis of the numerical value data that is output from the feature amount calculation unit 54 and relates to the first feature amount. Specifically, the determination unit 56 determines the quality of the fertilized egg F by applying the numerical value data relating to the first feature amount to a learned model (refer to Fig. 18). In addition, the determination unit 56 outputs the determination result to the display control unit 58 and the fertilized egg information database 59. The determination result output to the fertilized egg information database 59 is stored in the fertilized egg information database 59.

### (Step S07: Predicted Value Calculation)

The prediction unit 57 calculates a predicted value relating to the fertilized egg F on the basis of at least one of the numeral value data relating to the shape variation (state variation) or the numerical value data relating to the first feature amount which is output from the feature amount calculation unit 54. Specifically, the prediction unit 57 calculates at least one of a hatching rate, an implantation rate, a pregnancy rate, a conception rate, an abortion rate, a birth weight, a birth rate, or a breeding value as a predicted value relating to the fertilized egg F by applying at least one of the numerical value data relating to the shape variation (state variation) or the numerical value data relating to the first feature amount to the learned model (refer to Fig. 18). In addition, the prediction unit 57 outputs the predicted value to the display control unit 58 and the fertilized egg information database 59. The predicted value output to the fertilized egg information database 59 is stored in the fertilized egg information database 59.

### (Step S08: Quality Result Display)

The display control unit 58 causes the display device 60 as a web dashboard to display the first and second time lapses G1 and G2 (observation images) acquired from the image acquisition unit 51 and the image processing unit 52, an image after image processing (a fertilized egg recognition image, a motion vector image, a movement amount heat map image, and the like) which is acquired from the recognition unit 53, the state variation and the feature amount which are acquired from the feature amount calculation unit 54, the quality result of the fertilized egg F which is acquired from the determination unit 56, the predicted value acquired from the prediction unit 57, a growth stage code corresponding to a growth stage of the fertilized egg F, various images or various pieces of quality information which are read out from the fertilized egg information database 59, or the like.

According to this, a user can select a fertilized egg F before transplantation with high accuracy by collectively considering the observation image relating to the fertilized egg F, the fertilized egg recognition image, the motion vector image, the movement amount heat map image, the state variation, the feature amount, the quality result, the prediction value, and the like. Note that, the display control unit 58 may cause the display device 60 to also display position information of the well W in which the fertilized egg F is accommodated, imaging date and time, imaging conditions, or the like in addition the above-described factors.

### [Operation]

Recently, in fields of infertility treatment, animal husbandry, and the like, quality of cells (fertilized eggs) to be transplanted is an important factor that determines transplantation outcome. In selection of cells to be transplanted, typically, growth or quality of cells is determined in accordance with a morphological opinion by using an optical microscope, an image processing device, or the like.

However, with regard to quality evaluation of fertilized eggs before transplantation, in the morphological evaluation method as described above, skill is required, and it tends to be subjective. According to this, a quantitative and highly objective evaluation method is desired, and there is a demand for a method of evaluating the quality of fertilized eggs not only in a morphological manner but also in a multifaceted manner.

In consideration of such circumstances, in the information processing device 50 according to this embodiment, the quality of the fertilized egg F before transplantation is evaluated by using quality information in which the feature amount based on the shape variation of the fertilized egg F, and the quality result of the fertilized egg F which is obtained in accordance with the morphological opinion are correlated with each other. According to this, multifaceted quality evaluation in consideration of the morphological opinion of the fertilized egg F and the shape variation of the fertilized egg F can be performed, and it is possible to evaluate the fertilized egg F as an observation target with high accuracy.

In addition, in the information processing device 50 of this embodiment, it is possible to automatically calculate the state variation, the feature amount, or the like which relates to the fertilized egg F from an image of the fertilized egg F, and thus efficiency of evaluating the quality of the fertilized egg F is significantly improved in comparison to evaluation according to a morphological opinion obtained by confirming images of the fertilized egg F sheet by sheet by the embryologist in the related art.

### [Modification Example]

In the first embodiment, the feature amount calculation unit 54 calculates the number of times of contraction, the contraction diameter, the contraction speed, the contraction time, the contraction interval, the contraction strength, and the contraction frequency which relate to the fertilized egg F as the first feature amount, but there is no limitation thereto. The feature amount calculation unit 54 may calculate the number of times of expansion, an expansion diameter, an expansion speed, an expansion time, an expansion interval, expansion strength, an expansion frequency, and the like in accordance with an expansion phenomenon of the fertilized egg F as the first feature amount.

In addition, in the first embodiment, the determination unit 56 determines the quality of the fertilized egg F on the basis of the numerical value data that is output from the feature amount calculation unit 54 and relates to the first feature amount, but there is no limitation thereto. For example, the determination unit 56 may determine the quality of the fertilized egg F on the basis of any one or both of the numerical value data relating to the shape variation, and the numerical value data relating to the first feature amount which are output from the feature amount calculation unit 54.

### <Second Embodiment>

Next, a method of evaluating the quality of the fertilized egg F in an information processing device 50 according to a second embodiment of the present technology will be described appropriately with reference to Fig. 7. The information processing device 50 according to the second embodiment can execute the following steps in addition to the evaluation method described in the first embodiment. Note that, description of the same step as in the first embodiment will be omitted.

### [Fertilized Egg Quality Evaluation Method]

### (Step S03: Image Processing)

The recognition unit 53 performs predetermined image processing with respect to the second time lapse image G2 acquired from the image processing unit 52. The second time lapse image G2 that is subjected to the image processing by the recognition unit 53 is output to the feature amount calculation unit 54 and the fertilized egg information database 59, and the second time lapse image G2 is stored in the fertilized egg information database 59.

For example, the recognition unit 53 forms a mask region conforming to a shape of the fertilized egg F with respect to each image that constitutes the second time lapse image G2. According to this, an analysis region (recognition region) of the fertilized egg F in the second time lapse image becomes clear, and thus a position of the fertilized egg F in the well W is recognized.

### (Step S04: State Variation Calculation)

The feature amount calculation unit 54 performs predetermined analysis processing with respect to the second time lapse image G2 output from the recognition unit 53 to calculate a time-series variation of a relative position of the fertilized egg F with respect to the well W, and outputs numerical value data relating to the variation of the relative position to the imaging control unit 55, the determination unit 56, the prediction unit 57, the display control unit 58, and the fertilized egg information database 59. The numerical value data that is output to the fertilized egg information database 59 and relates to the variation of the relative position is stored in the fertilized egg information database 59.

In previous step S03, the feature amount calculation unit 54 calculates a difference value between a mask region of one image and a mask region of another image with respect to mask regions formed in respective images which constitute the second time lapse image G2. That is, the feature amount calculation unit 54 calculates a difference value between frames of only a mask region conforming to a shape of the fertilized egg F, and calculates a variation of the relative position of the fertilized egg F on the basis of the difference value.

According to this, occurrence of noise or erroneous detection due to calculation of the difference value between frames in the entirety of the images which constitute the second time lapse image G2 is suppressed, and thus it is possible to accurately calculate the variation of the relative position of the fertilized egg F with respect to the well W or a second feature amount to be described later.

With respect to the fertilized egg F in the well W, the feature amount calculation unit 54 calculates a time-series variation of a position in the X-axis direction (a variation of an X-coordinate position) and a time-series variation of a position in the Y-axis direction (a variation of a Y-coordinate position) as the variation of the relative position of the fertilized egg F with respect to the well W. According to this, when illustrating the variations with a graph or the like, it is possible to confirm the time-series variation of the relative position of the fertilized egg F in the well W.

### (Step S05: Feature Amount Calculation)

Next, the feature amount calculation unit 54 performs predetermined analysis processing with respect to the calculated variation of the relative position to calculate the second feature amount relating to the fertilized egg F, and outputs numerical value data relating to the second feature amount to the imaging control unit 55, the determination unit 56, the prediction unit 57, the display control unit 58, and the fertilized egg information database 59. The numerical value data that is output to the fertilized egg information database 59 and relates to second feature amount is stored in the fertilized egg information database 59.

In addition, the numerical value data that is output to the fertilized egg information database 59 and relates to the second feature amount is correlated with the second quality data (quality data in which the numerical value data relating to the first feature amount and the first quality data are correlated with each other) that is stored in the fertilized egg information database 59 in advance, and is stored in the fertilized egg information database 59 as third quality data.

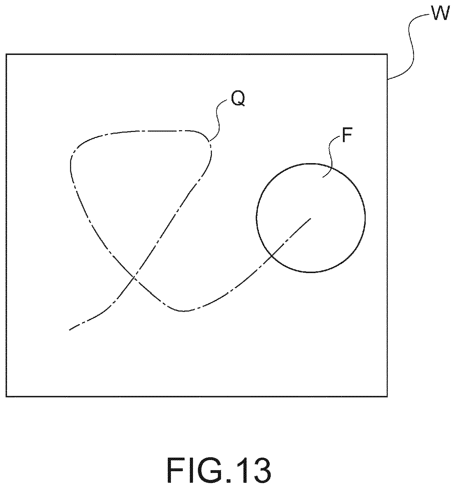

Fig. 13 is a view in which the second feature amount (movement trajectory) calculated by analyzing the variation of the relative position of the fertilized egg F accommodated in the well W is visualized. The feature amount calculation unit 54 calculates at least one of a central coordinate, a movement amount, a momentum, a movement distance (trajectory length), a movement speed, movement acceleration, or a movement trajectory of the fertilized egg F as the second feature amount. According to this, it is possible to confirm movement performance of the fertilized egg F in the well W by visualizing these values as illustrated in Fig. 13. Note that, in the example illustrated in Fig. 13, a curved line Q corresponds to the movement trajectory of the fertilized egg F in the well W.

### (Step S06: Quality Determination)

The determination unit 56 determines the quality (a growth situation, a quality rank, or the like) of the fertilized egg F on the basis of at least one of the numerical value data relating to the first feature amount or the numerical value data relating to the second feature amount which is output from the feature amount calculation unit 54. Specifically, the determination unit 56 determines the quality of the fertilized egg F by applying at least one of the numerical value data relating to the first feature amount or the numerical value data relating to the second feature amount to a learned model (refer to Fig. 18). In addition, the determination unit 56 outputs the determination result to the display control unit 58 and the fertilized egg information database 59. The determination result output to the fertilized egg information database 59 is stored in the fertilized egg information database 59.

### (Step S07: Predicted Value Calculation)

The prediction unit 57 calculates a predicted value relating to the fertilized egg F on the basis of at least one of the shape variation (state variation), the numerical value data relating to the first feature amount, or the numerical value data relating to the second feature amount which is output from the feature amount calculation unit 54. Specifically, the prediction unit 57 calculates at least one of a hatching rate, an implantation rate, a pregnancy rate, a conception rate, an abortion rate, a birth weight, a birth rate, or a breeding value as a predicted value relating to the fertilized egg F by applying at least one of the numerical value data relating to the shape variation (state variation), the numerical value data relating to the first feature amount, or the numerical value data relating to the second feature amount to the learned model (refer to Fig. 18). In addition, the prediction unit 57 outputs the predicted value to the display control unit 58 and the fertilized egg information database 59. The predicted value output to the fertilized egg information database 59 is stored in the fertilized egg information database 59.

### [Modification Example]

In the second embodiment, the determination unit 56 determines the quality of the fertilized egg F on the basis of at least one of the numerical value data relating to the first feature amount or the numerical value data relating to the second feature amount which is output from the feature amount calculation unit 54, but there is no limitation thereto. For example, the determination unit 56 may determine the quality of the fertilized egg F on the basis of any one or all of the numerical value data relating to the shape variation, the numerical value data relating to the relative position, the numerical value data relating to the first feature amount, and the numerical value data relating to the second feature amount which are output from the feature amount calculation unit 54.

### <Third Embodiment>

Next, a method of evaluating the quality of the fertilized egg F in an information processing device 50 according to a third embodiment of the present technology will be described appropriately with reference to Fig. 7. The information processing device 50 according to the third embodiment can execute the following steps in addition to the evaluation method described in the first and second embodiments. Note that, description of the same step as in the first and second embodiments will be omitted.

### [Fertilized Egg Quality Evaluation Method]

### (Step S03: Image Processing)

The recognition unit 53 performs predetermined image processing with respect to the second time lapse image G2 acquired from the image processing unit 52. The second time lapse image G2 that is subjected to the image processing by the recognition unit 53 is output to the feature amount calculation unit 54 and the fertilized egg information database 59, and the second time lapse image G2 is stored in the fertilized egg information database 59.

For example, the recognition unit 53 forms a mask region conforming to a shape of the fertilized egg F with respect to each image that constitutes the second time lapse image G2. According to this, an analysis region (recognition region) of the fertilized egg F in the second time lapse image G2 becomes clear, and thus a shape of cells inside the fertilized egg F can be accurately recognized.

### (Step S04: State Variation Calculation)

The feature amount calculation unit 54 performs predetermined analysis processing with respect to the second time lapse image G2 output from the recognition unit 53 to calculate a time-series variation of a macro movement amount of cells inside the fertilized egg F, and outputs numerical value data relating to the variation of the movement amount to the imaging control unit 55, the determination unit 56, the prediction unit 57, the display control unit 58, and the fertilized egg information database 59.

In previous step S03, the feature amount calculation unit 54 calculates a difference value between a mask region of one image and a mask region of another image with respect to mask regions formed in respective images which constitute the second time lapse image G2. That is, the feature amount calculation unit 54 calculates a difference value between frames of only a mask region conforming to a shape of the fertilized egg F, and calculates a variation of the movement amount of cells inside the fertilized egg F on the basis of the difference value.

According to this, occurrence of noise or erroneous detection due to calculation of the difference value between frames in the entirety of the images which constitute the second time lapse image G2 is suppressed, and thus it is possible to accurately calculate the variation of the movement amount of the cells inside the fertilized egg F.

The numerical value data that is output to the fertilized egg information database 59 and relates to the variation of the movement amount of the cells inside the fertilized egg F is stored in the fertilized egg information database 59, is correlated with the third quality data (quality data in which the numerical value data relating to the first feature amount, the numerical value data relating to the second feature amount, and the first quality data are correlated with each other) that is stored in the fertilized egg information database 59 in advance, and is stored in the fertilized egg information database 59 as fourth quality data.

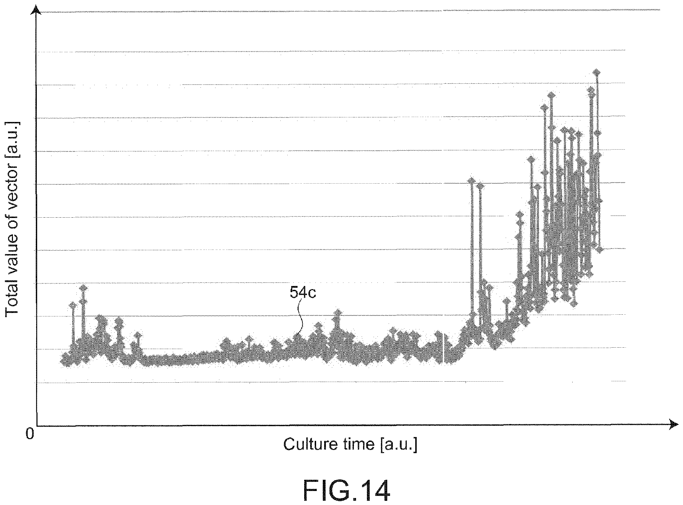

Fig. 14 is a graph illustrating a variation (a total value of a velocity vector) of the movement amount of the cells inside the fertilized egg F with respect to a culture time. The feature amount calculation unit 54 calculates at least one of time-series variations of a minimum speed, a maximum speed, maximum acceleration, an average speed, average acceleration, a central value, and a standard deviation of a motion vector, a total value of a velocity vector, and a total value of an acceleration vector as the variation of the movement amount of cells inside the fertilized egg F. According to this, when illustrating the variations with a graph or the like as illustrated in Fig. 14, in a case where an external shape variation of the fertilized egg F is small, it is possible to perform performance evaluation of the cells inside the fertilized egg F.

### (Step S06: Quality Determination)

The determination unit 56 determines the quality (a growth situation, a quality rank, or the like) of the fertilized egg F on the basis of at least one of the numerical value data relating to the first feature amount, the numerical value data relating to the second feature amount, or the numerical value data relating to the time-series variation of the movement amount of the cells inside the fertilized egg F which is output from the feature amount calculation unit 54. Specifically, the determination unit 56 determines the quality of the fertilized egg F by applying at least one of the numerical value data relating to the first feature amount, the numerical value data relating to the second feature amount, or the numerical value data relating to the time-series variation of the movement amount of the cells inside the fertilized egg F to the learned model (refer to Fig. 18). In addition, the determination unit 56 outputs the determination result to the display control unit 58 and the fertilized egg information database 59. The determination result output to the fertilized egg information database 59 is stored in the fertilized egg information database 59.

### (Step S07: Predicted Value Calculation)

The prediction unit 57 calculates a predicted value relating to the fertilized egg F on the basis of at least one of the numerical value data relating to the shape variation (state variation), the numerical value data relating to the first feature amount, the numerical value data relating to the second feature amount, or the numerical value data relating to the time-series variation of the movement amount of the cells inside the fertilized egg F which is output from the feature amount calculation unit 54. Specifically, the prediction unit 57 calculates at least one of a hatching rate, an implantation rate, a pregnancy rate, a conception rate, an abortion rate, a birth weight, a birth rate, or a breeding value as a predicted value relating to the fertilized egg F by applying at least one of the numerical value data relating to the shape variation (state variation), the numerical value data relating to the first feature amount, the numerical value data relating to the second feature amount, or the numerical value data relating to the time-series variation of the movement amount of the cells inside the fertilized egg F to the learned model (refer to Fig. 18). In addition, the prediction unit 57 outputs the predicted value to the display control unit 58 and the fertilized egg information database 59. The predicted value output to the fertilized egg information database 59 is stored in the fertilized egg information database 59.

### [Modification Example]

In the third embodiment, the determination unit 56 determines the quality of the fertilized egg F on the basis of at least one of the first feature amount, the second feature amount, or the numerical value data relating to the variation of the movement amount of the cells inside the fertilized egg F which is output from the feature amount calculation unit 54, but there is no limitation thereto. For example, the determination unit 56 may determine the quality of the fertilized egg F on the basis of any one or all of the numerical value data relating to the shape variation, the numerical value data relating to the variation of the relative position, the numerical value data relating to the first feature amount, the numerical value data relating to the second feature amount, and the numerical value data relating to the variation of the movement amount of the cells inside the fertilized egg F which are output from the feature amount calculation unit 54.

### <Fourth Embodiment>

Next, a method of evaluating the quality of the fertilized egg F in an information processing device 50 according to a fourth embodiment of the present technology will be described appropriately with reference to Fig. 7. The information processing device 50 according to the fourth embodiment can execute the following steps in addition to the evaluation method described in the first to third embodiments. Note that, description of the same step as in the first to third embodiment will be omitted.

### [Fertilized Egg Quality Evaluation Method]

### (Step S05: Feature Amount Calculation)

The image acquisition unit 51 acquires the first time lapse image G1 output from the imaging unit 21, and outputs the first time lapse image G1 to the feature amount calculation unit 54. The feature amount calculation unit 54 performs predetermined analysis processing with respect to the first time lapse image G1 acquired from the image acquisition unit 51 to calculate a third feature amount relating to the fertilized egg F, and outputs numerical value data relating to the third feature amount to the imaging control unit 55, the determination unit 56, the prediction unit 57, and the fertilized egg information database 59. The numerical value data that is output to the fertilized egg information database 59 and relates to the third feature amount is stored in the fertilized egg information database 59.

In addition, the numerical value data that is output to the fertilized egg information database 59 and relates to the third feature amount is correlated with the fourth quality data (quality data in which the numerical value data relating to the first feature amount, the numerical value data relating to the second feature amount, the first quality data, and the numerical value data relating to the variation of the movement amount of the cells inside the fertilized egg F are correlated with each other) that is stored in the fertilized egg information database 59 in advance, and is stored in the fertilized egg information database 59 as fifth quality data.

Here, the third feature amount is information of a characteristic portion of an image of the fertilized egg F, and examples thereof include a size, a shape, a sphericity, and a cleavage number (rate) of the fertilized egg, a shape of each blastomere and symmetry thereof, fragmentation, and a size and a shape of ICM which are calculated on the basis of various growth stages of the fertilized egg F.

### (Step S06: Quality Determination)

The determination unit 56 determines the quality (a growth situation, a quality rank, or the like) of the fertilized egg F on the basis of at least one of the numerical value data relating to the first feature amount, the numerical value data relating to the second feature amount, the numerical value data relating to the third feature amount, or the numerical value data relating to the time-series variation of the movement amount of the cells inside the fertilized egg F which is output from the feature amount calculation unit 54. Specifically, the determination unit 56 determines the quality of the fertilized egg F by applying at least one of the numerical value data relating to the first feature amount, the numerical value data relating to the second feature amount, the numerical value data relating to the third feature amount, or the numerical value data relating to the time-series variation of the movement amount of the cells inside the fertilized egg F to the learned model (refer to Fig. 18). At this time, a growth stage code corresponding to a growth stage is applied to the image of the fertilized egg F as an evaluation target.

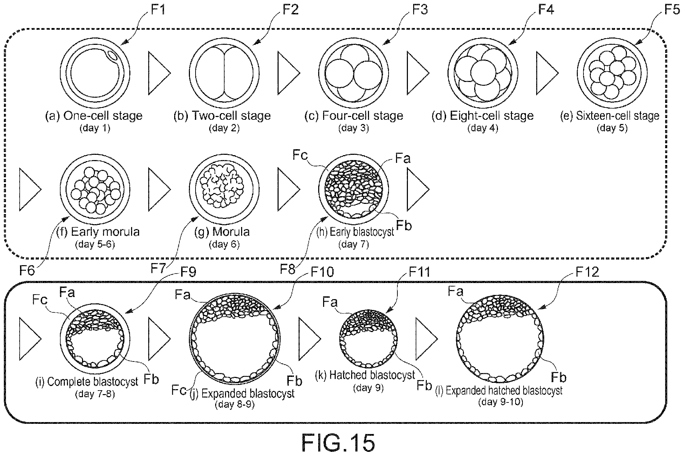

For example, the growth stage code is applied to the image of the fertilized egg F in correspondence with a growth stage of the fertilized egg F as follows. A growth stage of a one-cell stage F1 is set to a growth stage code 1, a growth stage from a two-cell stage F2 to a sixteen-cell stage F5 is set to a growth stage code 2, a growth stage of an early morula F6 is set to a growth stage code 3, a growth stage of a morula F7 is set to a growth stage code 4, a growth stage of an early blastocyst F8 is set to a growth stage code 5, a growth stage of a complete blastocyst F9 is set to a growth stage code 6, a growth stage of an expanded blastocyst F10 is set to a growth stage code 7, a growth stage of a hatched blastocyst F11 is set to a growth stage code 8, and a growth stage of an expanded hatched blastocyst F12 is set to a growth stage code 9 (refer to Fig. 15).

The determination unit 56 outputs a determination result obtained by determining the quality of the fertilized egg F to the display control unit 58 and the fertilized egg information database 59. The determination result output to the fertilized egg information database 59 is stored in the fertilized egg information database 59.

### (Step S07: Predicted Value Calculation)

The prediction unit 57 calculates a predicted value relating to the fertilized egg F on the basis of at least one of the numerical value data relating to the shape variation (state variation), the numerical value data relating to the first feature amount, the numerical value data relating to the second feature amount, the numerical value data relating to the third feature amount, or the numerical value data relating to the time-series variation of the movement amount of the cells inside the fertilized egg F which is output from the feature amount calculation unit 54. Specifically, the prediction unit 57 calculates at least one of a hatching rate, an implantation rate, a pregnancy rate, a conception rate, an abortion rate, a birth weight, a birth rate, or a breeding value as a predicted value relating to the fertilized egg F by applying at least one of the numerical value data relating to the shape variation (state variation), the numerical value data relating to the first feature amount, the numerical value data relating to the second feature amount, the numerical value data relating to the third feature amount, or the numerical value data relating to the time-series variation of the movement amount of the cells inside the fertilized egg F to the learned model (refer to Fig. 18). In addition, the prediction unit 57 outputs the predicted value to the display control unit 58 and the fertilized egg information database 59. The predicted value output to the fertilized egg information database 59 is stored in the fertilized egg information database 59.

### [Modification Example]

In the fourth embodiment, the determination unit 56 determines the quality of the fertilized egg F on the basis of at least one of the numerical value data relating to the first feature amount, the numerical value data relating to the second feature amount, the numerical value data relating to the third feature amount, or the numerical value data relating to the variation of the movement amount of the cells inside the fertilized egg F which is output from the feature amount calculation unit 54, but there is no limitation thereto. For example, the determination unit 56 may determine the quality of the fertilized egg F on the basis of any one or all of the numerical value data relating to the shape variation, the numerical value data relating to the variation of the relative position, the pieces of numerical value data relating to the first to third feature amounts, and the numerical value data relating to the variation of the movement amount of the cells inside the fertilized egg F which are output from the feature amount calculation unit 54.

### <Fifth Embodiment>

Next, a method of evaluating the quality of the fertilized egg F in an information processing device 50 according to a fifth embodiment of the present technology will be described. First, in description of the quality evaluation method according to the fifth embodiment, a morphological variation due to growth of the fertilized egg will be described with reference to Fig. 15.

Fig. 15 illustrates a typical growth stage of the fertilized egg from a first day to a tenth day after fertilization. Fig. 15(a) illustrates a fertilized egg F1 in a one-cell stage at a first day after confirmation of fertilization. When reaching a second day after fertilization, as illustrated in Fig. 15(b), a fertilized egg F2 in a two-cell stage is obtained after division into two parts.

After the fertilized egg F smoothly grows, as illustrated in Fig. 15(c), Fig. 15(d), and Fig. 15(e), the number of cells increases as in a four-cell stage fertilized egg F3 at a third day after fertilization, in an eight-cell stage fertilized egg F4 at a fourth day after fertilization, and in a sixteen-cell stage fertilized egg F5 at a fifth day after fertilization in this order.

Then, cells come into close contact with each other, the fertilized egg becomes an early morula F6 at fifth to sixth days after fertilization as illustrated in Fig. 15(f), and becomes a morula F7 at a sixth day after fertilization as illustrated in Fig. 15(g). When the fertilized egg further grows, a gap occurs in a cytoplasm, a blastocoel is formed, and the fertilized egg becomes an early blastocyst F8 at a seventh day after fertilization as illustrated in Fig. 15(h).

When the blastocoel is enlarged, the fertilized egg becomes a complete blastocyst F9 at seventh to eight days after fertilization as illustrated in Fig. 15(i). When reaching a blastocyst growth stage (after F8), it is possible to identify an inner cell mass (hereinafter, referred to as "ICM") Fa that becomes a future fetus, and a trophectoderm Fb.

In the growth stages of the early blastocyst F8 and the complete blastocyst F9, a transparent zone Fc that forms an external shape of the fertilized egg is recognized. In addition, the transparent zone Fc becomes thin, and the fertilized egg becomes an expanded blastocyst F10 at eight to ninth days after fertilization. The fertilized egg becomes a hatched blastocyst F11 in which the blastocyst is hatched from the transparent zone Fc at a ninth day after fertilization, and becomes an expanded hatched blastocyst F12 at ninth to tenth days after fertilization.

Typically, there is known that a lag-phase (rest period) in which active proliferation of cells is stopped is present in the fertilized egg that undergoes the growth course as described above. Here, recently, it was found that the lag-phase occurs in the course of division from the four-cell stage (F3) to the eight-cell stage (F4), and the greater the number of lag-phase initiation cells is, the earlier an initiation time is, and the shorter a stage is, the higher developmental potency (a pregnancy rate and the like) of the fertilized egg after transplantation is (refer to http://www.naro.affrc.go.jp/project/results/laboratory/ niah/1999/niah99-021.html).

Due to the above-described circumstances, in fields of infertility treatment, animal husbandry, and the like, the lag-phase of the fertilized egg has attracted attention as an important index for identifying a fertilized egg with high developmental potency.

Here, in the fifth embodiment, a method of determining the lag-phase of the fertilized egg F, and of predicting the developmental potency relating to the fertilized egg F after transplantation from the lag-phase will be described appropriately with reference to Fig. 7. Note that, the information processing device 50 according to the fifth embodiment executes the following steps in addition to the evaluation method described in the first to fourth embodiments, and description of the same step as in the first to fourth embodiments will be omitted.

### [Fertilized Egg Quality Evaluation Method]

### (Step S06: Quality Determination)

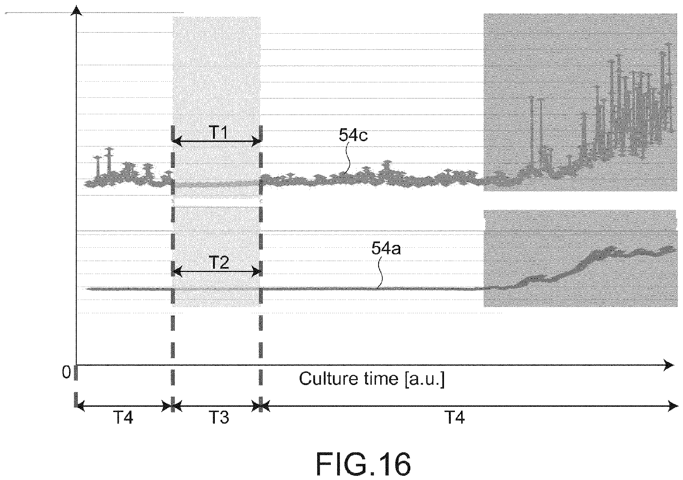

Fig. 16 is a view in which a graph 54a illustrating a time-series shape variation (diameter variation) of the fertilized egg F with respect to a culture time, and a graph 54c illustrating a time-series variation (a total value of a velocity vector) of a movement amount of cells inside the fertilized egg F are shown in combination.

The determination unit 56 reads out numerical value data relating to a variation (refer to the third embodiment) of a total value of the velocity vector of cells inside the fertilized egg F from the fertilized egg information database 59, and performs predetermined analysis processing with respect to the numerical value data to detect a first period T1 in which the variation of the total value of the velocity vector per unit culture time is approximately zero.

Next, the determination unit 56 reads out numerical value data relating to a variation (refer to the first embodiment) of a diameter of the fertilized egg F in which the first period T1 is detected from the fertilized egg information database 59, and performs predetermined analysis processing with respect to the numerical value data to detect a second period T2 in which a time-series variation of the diameter per unit culture time is approximately zero.

Next, the determination unit 56 determines a lag-phase period T3 (activity rest stage) of the fertilized egg F on the basis of the first and second periods T1 and T2 with respect to the fertilized egg F in which the first and second periods T1 and T2 are detected. That is, the determination unit 56 determines a culture period of the fertilized egg F that is the first period T1 and the second period T2 as the lag-phase period T3 of the fertilized egg F.

Next, the determination unit 56 correlates the numerical value data relating to the variation of the diameter of the fertilized egg F in the lag-phase period T3 and the first quality data (a growth situation, the number of cells, a culture elapse time, and the like), which is evaluated in previous step S02, of the fertilized egg F in the lag-phase period T3 with each other to generate sixth quality data. In addition, the determination unit 56 outputs the sixth quality data to the prediction unit 57 and the fertilized egg information database 59. The sixth quality data output to the fertilized egg information database 59 is stored in the fertilized egg information database 59.

### (Step S07: Predicted Value Calculation)

The prediction unit 57 calculates a predicted value relating to the fertilized egg F on the basis of the sixth quality data output from the determination unit 56. Specifically, the prediction unit 57 calculates at least one of a hatching rate, an implantation rate, a pregnancy rate, a conception rate, an abortion rate, a birth weight, a birth rate, or a breeding value as the predicted value relating to the fertilized egg F by applying the sixth quality data to the learned model (refer to Fig. 18). In addition, the prediction unit 57 outputs the predicted value to the display control unit 58 and the fertilized egg information database 59. The predicted value output to the fertilized egg information database 59 is stored in the fertilized egg information database 59.

### [Operation]

In the information processing device 50 according to the fifth embodiment, the lag-phase period T3 of the fertilized egg F, and the predicted value based on the lag-phase period T3 can be automatically calculated. According to this, efficiency of a process of selecting a fertilized egg F of which the developmental potency after transplantation is predicted to be high is significantly improved.

### [Modification Example]

In the fifth embodiment, the determination unit 56 determines the lag-phase period T3 of the fertilized egg F on the basis of the first and second periods T1 and T2, but there is no limitation thereto. For example, the determination unit 56 may determine any one or both of the lag-phase period T3 of the fertilized egg F and an active period T4 other than the lag-phase period T3 on the basis of the first and second periods T1 and T2.

In this case, the determination unit 56 may calculate predicted values (the hatching rate, the implantation rate, the pregnancy rate, the conception rate, the abortion rate, the birth weight, the birth rate, and the breeding value) which relate to the fertilized egg F on the basis of any one or both of the lag-phase period T3 and the active period T4, and the first quality data relating to the fertilized egg F in the periods by the same method as in the quality evaluation method. In addition, the lag-phase period T3 of the fertilized egg F may be determined on the basis of any one of the first and second periods T1 and T2.

In addition, in the fifth embodiment, the determination unit 56 determines a state of the fertilized egg F in which the variation of the diameter of the fertilized egg F per unit culture time is approximately zero and the variation of the total value of the velocity vector per unit culture time is approximately zero as the lag-phase period T3, but there is no limitation thereto. For example, the determination unit 56 may determine a state of the fertilized egg F in which the variation of the diameter of the fertilized egg F per unit culture time and the variation of the total value of the velocity vector per unit culture time are in predetermined ranges as the lag-phase period T3.

In addition, in the fifth embodiment, the determination unit 56 detects the first period T1 on the basis of the numerical value data relating to the time-series variation of the total value of the velocity vector of the fertilized egg F, but there is no limitation thereto. The determination unit 56 may detect the first period T1 on the basis of numerical value data relating to time-series variations (refer to the third embodiment) of a minimum speed, a maximum speed, maximum acceleration, an average speed, average acceleration, a central value, a standard deviation, and the total value of the velocity vector in addition to the time-series variation of the total value of the velocity vector of the fertilized egg F.

In addition, in the fifth embodiment, the determination unit 56 detects the second period T2 on the basis of the numerical value data relating to the variation of the diameter of the fertilized egg F, but there is no limitation thereto. The determination unit 56 may detect the second period T2 on the basis of the numerical value data relating to a variation (refer to the first embodiment) of an area, a volume, or a roundness in addition to the numerical value data relating to the variation of the diameter of the fertilized egg F.

### <With Regard to Quality Determination of Fertilized Egg and Calculation of Predicted Value>

The information processing device 50 of the present technology performs quality determination and calculation of a predicted value with respect to the fertilized egg F by using so-called specialized artificial intelligence (AI) that substitutes for user's artificial work. Fig. 18 is a block diagram simply illustrating a procedure of typical specialized AI.

With regard to a general structure of the specialized AI, the specialized AI is a mechanism in which arbitrary input data is applied to a learned model obtained by causing an algorithm that functions as a learning program to learn learning data, and a result is obtained.

Here, in the present technology, the numerical value data relating to the shape variation (state variation), the numerical value data relating to the time-series variation of the relative position of the fertilized egg F with respect to the well W, the numerical value data relating to the time-series variation of the movement amount of the cells inside the fertilized egg F, the pieces of numerical value data relating to the first to third feature amount, and the first quality data to the sixth quality data which are stored in the fertilized egg information database 59 correspond to "learning data" in Fig. 18.

In addition, the numerical value data relating to the shape variation (state variation), the numerical value data relating to the time-series variation of the relative position of the fertilized egg F with respect to the well W, the numerical value data relating to the time-series variation of the movement amount of the cells inside the fertilized egg F, and the pieces of numerical value data relating to the first to third feature amounts which are output from the feature amount calculation unit 54 to the determination unit 56 and the prediction unit 57, the first quality data input to the input unit 70, and the sixth quality data that is input from the determination unit 56 to the prediction unit 57 correspond to the "input data" in Fig. 18.

In addition, for example, an "algorithm" in Fig. 18 is a machine learning algorithm. The kind of the machine learning algorithm is not particularly limited, and examples thereof include algorithms using neural networks such as a recurrent neural network (RNN), a convolutional neural network (CNN), a multilayer perceptron (MLP), or the like, and the machine learning algorithm may be an arbitrary algorithm that executes a supervised learning method, a unsupervised learning method, a semi-supervised learning method, a reinforced learning method, or the like.

In addition, the determination result obtained through determination of the quality of the fertilized egg F by the determination unit 56, and the predicted value calculated by the prediction unit 57 correspond to "result" in Fig. 18.

### <Sixth Embodiment>

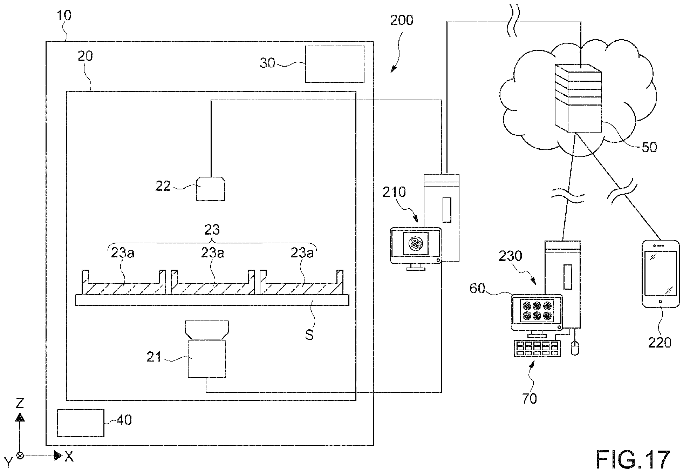

Next, an observation system 200 according to a sixth embodiment of the present technology will be described. Fig. 17 is a view schematically illustrating a configuration example of the observation system 200 according to the sixth embodiment of the present technology. Hereinafter, the same reference numeral will be given to the same configuration as in the first embodiment, and detailed description thereof will be omitted.

The observation system 200 according to the sixth embodiment has a configuration of performing quality analysis of the fertilized egg F by processing, accumulation, and machine learning of a captured image on a cloud side different from a location, in which an observation device 20 including an imaging unit 21 that captures an image of the fertilized egg F is provided, through a network. That is, an information processing device 50 according to this embodiment is configured as a cloud server.

As illustrated in Fig. 17, the observation system 200 includes an incubator 10, the observation device 20, a humidity/temperature/gas control unit 30, a detection unit 40, the information processing device 50, and a gateway terminal PC 210. The information processing device 50 according to the sixth embodiment is connected to the gateway terminal PC 210 through a network. In addition, a portable terminal 220 and a PC 230 are connected to the information processing device 50 through the network.

The gateway terminal PC 210 is connected to the observation device 20, receives the first and second time lapse images G1 and G2 from the imaging unit 21, and outputs the images to the information processing device 50 through the network. In addition, the gateway terminal PC 210 stores the first and second time lapse images G1 and G2, and receives the first and second lapse images G1 and G2 from the information processing device 50 through the network and displays the images through an operation by a user.

Hereinbefore, the embodiments of the present technology have been described. However, the present technology is not limited to the above-described embodiments, and various modifications can be made.

For example, in the observation systems 100 and 200, step S01 is repeated continuously or for every arbitrary period, for example, for every fifteen minutes or for every other day, and the quality of the fertilized egg F is evaluated by using an image obtained in the process, but there is no limitation thereto. The observation systems 100 and 200 of the present technology may acquire an image in real time in correspondence with necessity, or the display device 60 may be caused to display an image of the fertilized egg F to frequently perform observation and evaluation.

In addition, the fertilized egg F that is observed by the observation systems 100 and 200 of the present technology is typically derived from a cow. However, there is no limitation thereto, and the fertilized egg F may be a fertilized egg derived from a domestic animal such as a mouse, a pig, a dog, and a cat, or a fertilized egg derived from human beings.

In addition, in this specification, the "fertilized egg" conceptually includes at least a single cell, and an aggregate of a plurality of cells. Here, the single cell or the aggregate of a plurality of cells relates to cells which are observed in one stage or a plurality of stages in a growth course of fertilized eggs including an oocyte, an ovum, a fertilized egg (a fertile ovum or a zygote), a blastocyst, and an embryo.

In addition, the present technology is applicable to arbitrary cells such as unfertilized egg cell (ovum) and morula in an animal husbandry field or the like, biological materials such as a stem cell, an immunocyte, and a cancer cell which are taken out from living matters in fields of a regenerative medicine, a pathology and biology, and a gene editing technology, and the like.

Note that, the present technology can employ the following configurations.
(1) An information processing device, including:
   an image acquisition unit that acquires a time lapse image of a fertilized egg imaged on a time-series basis;
   a feature amount calculation unit that calculates a time-series state variation of the fertilized egg; and
   a determination unit that determines any one or both of an active period and a rest period of the fertilized egg on the basis of the state variation.
(2) The information processing device according to (1), in which
   the feature amount calculation unit calculates a variation of a movement amount inside the fertilized egg as the state variation.
(3) The information processing device according to (1) or (2), in which
   the feature amount calculation unit calculates a shape variation of the fertilized egg as the state variation.
(4) The information processing device according to (3), in which
   the feature amount calculation unit calculates a diameter variation of the fertilized egg as the shape variation.
(5) The information processing device according to (3) or (4), in which
   the determination unit determines, as the rest period, a state of the fertilized egg in which the variation of the movement amount per unit time and the shape variation per unit time are in predetermined variation amount ranges.
(6) The information processing device according to any one of (3) to (5), in which
   the determination unit determines, as the rest period, a state of the fertilized egg in which the shape variation per unit time is approximately zero and the variation of the movement amount per unit time is approximately zero.
(7) The information processing device according to any one of (1) to (6), further including:
   a prediction unit that calculates, on the basis of any one or both of the active period and the rest period, at least one of a hatching rate, an implantation rate, a pregnancy rate, a conception rate, an abortion rate, a birth weight, a birth rate, or a breeding value related to the fertilized egg.
(8) The information processing device according to (7), in which
   the prediction unit calculates at least one of the hatching rate, the implantation rate, the pregnancy rate, the conception rate, the abortion rate, the birth weight, the birth rate, or the breeding value by using a learned model obtained by causing a machine learning algorithm to learn learning data.
(9) An information processing method, including:
   acquiring a time lapse image of a fertilized egg imaged on a time-series basis;
   calculating a time-series state variation of the fertilized egg; and
   determining any one or both of an active period and a rest period of the fertilized egg on the basis of the state variation.
(10) A program that causes an information processing device to execute:
   a step of acquiring a time lapse image of a fertilized egg imaged on a time-series basis;
   a step of calculating a time-series state variation of the fertilized egg; and
   a step of determining any one or both of an active period and a rest period of the fertilized egg on the basis of the state variation.
(11) An observation system, including:
   an imaging unit that images a fertilized egg on a time-series basis; and
   an information processing device including an image acquisition unit that acquires a time lapse image captured by the imaging unit, a feature amount calculation unit that calculates a time-series state variation of the fertilized egg, and a determination unit that determines any one or both of an active period and a rest period of the fertilized egg on the basis of the state variation.

### Reference Signs List

- 100, 200: observation system
- 10: incubator
- 20: observation device
- 21: imaging unit
- 22: light source
- 23: culture container group
- 23a: culture container
- 30: humidity/temperature/gas control unit
- 40: detection unit
- 50: information processing device
- 51: image acquisition unit
- 52: image processing unit
- 53: recognition unit
- 54: feature amount calculation unit
- 55: imaging control unit
- 56: determination unit
- 57: prediction unit
- 58: display control unit
- 59: fertilized egg information database
- 60: display device
- 70: input unit
- F: fertilized egg
- W: well

## Claims

1. An information processing device, comprising:
an image acquisition unit that acquires a time lapse image of a fertilized egg imaged on a time-series basis;
a feature amount calculation unit that calculates a time-series state variation of the fertilized egg; and
a determination unit that determines any one or both of an active period and a rest period of the fertilized egg on a basis of the state variation.

2. The information processing device according to claim 1, wherein
the feature amount calculation unit calculates a variation of a movement amount inside the fertilized egg as the state variation.

3. The information processing device according to claim 2, wherein
the feature amount calculation unit further calculates a shape variation of the fertilized egg as the state variation.

4. The information processing device according to claim 3, wherein
the feature amount calculation unit calculates a diameter variation of the fertilized egg as the shape variation.

5. The information processing device according to claim 3, wherein
the determination unit determines, as the rest period, a state of the fertilized egg in which the variation of the movement amount per unit time and the shape variation per unit time are in predetermined variation amount ranges.

6. The information processing device according to claim 5, wherein
the determination unit determines, as the rest period, a state of the fertilized egg in which the shape variation per unit time is approximately zero and the variation of the movement amount per unit time is approximately zero.

7. The information processing device according to claim 1, further comprising:
a prediction unit that calculates, on a basis of any one or both of the active period and the rest period, at least one of a hatching rate, an implantation rate, a pregnancy rate, a conception rate, an abortion rate, a birth weight, a birth rate, or a breeding value related to the fertilized egg.

8. The information processing device according to claim 7, wherein
the prediction unit calculates at least one of the hatching rate, the implantation rate, the pregnancy rate, the conception rate, the abortion rate, the birth weight, the birth rate, or the breeding value by using a learned model obtained by causing a machine learning algorithm to learn learning data.

9. An information processing method, comprising:
acquiring a time lapse image of a fertilized egg imaged on a time-series basis;
calculating a time-series state variation of the fertilized egg; and
determining any one or both of an active period and a rest period of the fertilized egg on a basis of the state variation.

10. A program that causes an information processing device to execute:
a step of acquiring a time lapse image of a fertilized egg imaged on a time-series basis;
a step of calculating a time-series state variation of the fertilized egg; and
a step of determining any one or both of an active period and a rest period of the fertilized egg on a basis of the state variation.

11. An observation system, comprising:
an imaging unit that images a fertilized egg on a time-series basis; and
an information processing device including an image acquisition unit that acquires a time lapse image captured by the imaging unit, a feature amount calculation unit that calculates a time-series state variation of the fertilized egg, and a determination unit that determines any one or both of an active period and a rest period of the fertilized egg on a basis of the state variation.
